# EUROPÄISCHE PATENTSCHRIFT

(11) **EP 2 900 334 B1**
(45) Veröffentlichungstag und Bekanntmachung des Hinweises auf die Patenterteilung: **16.08.2017**
(21) Anmeldenummer: 14723982.6
(22) Anmeldetag: 17.03.2014
(51) Int. Cl.: A61Q 11/00, A61K 8/34, A61K 8/37, A61K 8/92, A61K 8/97

(54) **ZUSAMMENSETZUNG FÜR DIE MUNDPFLEGE**
COMPOSITIONS FOR ORAL CAVITY CARE
COMPOSITIONS POUR LES SOINS DE LA CAVITÉ ORALE

(30) Priorität: 18.03.2013 DE 102013004521
(43) Veröffentlichungstag der Anmeldung: 05.08.2015
(73) Patentinhaber: theranovis GmbH & Co. KG, 55411 Bingen (DE)
(72) Erfinder: PATEL, Ashokkumar, Minnetrista, MN 55344 (US)
(74) Vertreter: Beyer, Carsten
(86) Internationale Anmeldenummer: PCT/DE2014/100091
(87) Internationale Veröffentlichungsnummer: WO 2014/146644

(56) Entgegenhaltungen:
- EP-A1- 1 886 662
- EP-A2- 1 954 831
- WO-A2-2006/027248
- WO-A2-2006/078699
- DE-A1- 10 350 929
- DE-A1-102010 031 263

## Beschreibung

Die vorliegende Erfindung betrifft eine Zusammensetzung für die Mundpflege, wobei die Zusammensetzung eine wässrige Zusammensetzung ist. Ferner betrifft die Erfindung Verwendungen der genannten Zusammensetzung. Schließlich sind ein Verfahren zur Lösung und/oder Entfernung von extrazellulären polymeren Substanzen (EPS) auf Zahnoberflächen und/oder Zahnbelag (Plaque) und/oder Zahnstein und ein Verfahren zur Modifikation der Pellikelschicht auf Zahnoberflächen, jeweils durch Anwenden der erfindungsgemäßen Zusammensetzung, angegeben.

Die zerstörerische Wirkung von Karies auf Zahnoberflächen und Zähne insgesamt ist gemeinhin bekannt. So leiden ungefähr 90% der erwachsenen Personen an Karies. Ferner sind Krankheiten, insbesondere entzündliche Veränderungen, des Zahnfleisches (Gingiva) weit verbreitet. Aus einer Gingivitis kann sich dabei rasch eine Parodontitis entwickeln. Ein ursächlicher Zusammenhang zwischen dem Vorhandensein von Zahnbelag (Plaque) und insbesondere von Zahnstein und verschiedenen Erkrankungen der Gingiva ist dabei allgemein anerkannt.

Als Basis für eine geeignete Prophylaxe gilt allgemein das zwei- oder mehrmals am Tag durchzuführende Zähneputzen, um eine unerwünschte Belagbildung auf den Zahnoberflächen und insgesamt einen bakteriellen Befall des Mundraums zumindest einzudämmen.

Allerdings lässt sich bereits wenige Minuten nach dem Zähneputzen die Neubildung der sog. Pellikelschicht auf den Zahnoberflächen nachweisen. Die Bezeichnungen "Pellikelschicht" und "Pellikel" werden hier und im Folgenden synonym verwendet. Dabei handelt es sich um einen Niederschlag aus Proteinen aus dem Speichel. Die Pellikel ist für die Zahngesundheit an sich wenig bedenklich. Allerdings siedeln sich im Folgenden auf der Pellikel weitere Mikroorganismen aus der Speichelflora an, was zu einem Dickenwachstum des entstandenen Biofilms führt.

Hierbei können sich auch schädliche Mikroorganismen ansiedeln. Dabei kann eine Matrix aus extrazellulären polymeren Substanzen (EPS) entstehen, welche verschiedenste Bakterienspezies auf der Zahnoberfläche einzubetten vermag. Die entstehende Zellschicht wird als Zahnbelag (Plaque) bezeichnet und fördert das Auftreten von Karies immens.

Aus der Plaque kann durch Aufnahme und Einlagerung anorganischer Stoffe (Mineralstoffe) aus dem Speichel Zahnstein entstehen. Dieser ist i.A. nicht mehr durch die Zahnbürste entfernbar. Der Zahnstein fördert das Auftreten der oben angeführten Erkrankungen der Gingiva.

Aus dem Stand der Technik sind Zusammensetzungen bekannt, deren Anwendung zumindest das Reinigen der Zahnoberflächen und so das Eindämmen oder die Entfernung von Plaque bewirken soll.

Aus DE 10 2008 033 105 A1 sowie DE 10 2008 039 681 A1 sind Mund- und Zahnpflege- und -reinigungsmittel bekannt, welche vorzugsweise in Form bekannter Zahnpasten ausgestaltet sind. Diese enthalten in einem fließfähigen Träger suspendierte Partikel, welche u.a. synthetische Materialien aufweisen können. Heutzutage werden vom Markt jedoch vermehrt Produkte mit natürlichen Inhaltsstoffen gefordert. Des Weiteren ist die Anwendung dieser bekannten Reinigungsmittel üblicherweise auf das Zähneputzen beschränkt, womit allerdings die oben beschriebene Neubildung eines Biofilms nach dem Putzen regelmäßig einhergeht. Schließlich ist das Zähneputzen nicht an jedem Ort und in unauffälliger Weise möglich.

DE 200 13 336 U1 beschreibt ein ölbasiertes Mund- und Zahnpflegegel, das bei der Prophylaxe und/oder Behandlung von entzündlichen Erkrankungen des Zahnbettes und/oder des Zahnhalses Anwendung finden soll. Dazu enthält das Gel eine Zubereitung aus verschiedenen Vitaminen. Allerdings ist in Bezug auf diese Zubereitung keine Wirkung auf unerwünschte Beläge auf Zahnoberflächen dargetan.

Der vorliegenden Erfindung liegt daher die Aufgabe zugrunde, zumindest einen der oben aufgeführten Nachteile zu lindern.

Des Weiteren liegt der Erfindung die Aufgabe zugrunde, einen Beitrag zur Lösung und/oder Entfernung von Pellikel und/oder Zahnbelag (Plaque) und/oder Zahnstein von Zahnoberflächen zu leisten.

Des Weiteren liegt der Erfindung die Aufgabe zugrunde, einen Beitrag zur antibakteriellen Behandlung des Mundraums, insbesondere von Zahnoberflächen und/oder der Gingiva, zu leisten.

Des Weiteren liegt der Erfindung die Aufgabe zugrunde, eine Zusammensetzung für die Mundpflege bereitzustellen, welche möglichst weitgehend oder vollständig aus natürlichen Inhaltsstoffen besteht.

Schließlich liegt der Erfindung die Aufgabe zugrunde, einen Beitrag zu einer einfachen Anwendung einer Zusammensetzung für die Mundpflege zu leisten.

Einen Beitrag zur Lösung mindestens einer der vorstehend genannten Aufgaben leistet eine Zusammensetzung für die Mundpflege mit den Merkmalen des Anspruchs 1.

Vorteilhafte Ausgestaltungen und Weiterbildungen ergeben sich aus den hierzu nachgeordneten Ansprüchen.

Im Rahmen der vorliegenden Erfindung hat sich gezeigt, dass die erfindungsgemäße Zusammensetzung für die Mundpflege eine überraschend gute Wirkung bei der Inhibierung, Reduktion und Auflösung (Eliminierung) von Biofilmen auf Zahnoberflächen zeigt. Die Zusammensetzung ist in überraschender Weise sehr gut dazu geeignet, die Ausbildung derartiger Biofilme auf Zahnoberflächen zu kontrollieren oder sogar gänzlich zu verhindern. Dabei werden einerseits die Pellikelbildung modifiziert und andererseits ggf. vorhandene extrazelluläre polymere Strukturen (EPS) aufgelöst. Die Neubildung genannter Biofilme wird auch nach der Anwendung nachhaltig gestört.

Die Anwendung der erfindungsgemäßen Zusammensetzung auf der gereinigten Zahnoberfläche beeinflusst und modifiziert die Pellikelbildung dergestalt, dass die Adhärenz von Bakterien nachhaltig reduziert wird. Des Weiteren ist die Fähigkeit der adhärenten Bakterien, sich zu teilen und die Zahnoberfläche zu kolonisieren, deutlich verringert bzw. gestört.

Folglich kann bereits die Entstehung von Zahnbelag (Plaque) kontrolliert oder sogar unterbunden werden. Schädliche Bakterien können sich nicht mehr an den Zahnoberflächen anlagern.

Es hat sich des Weiteren in überraschender Weise erwiesen, dass die erfindungsgemäße Zusammensetzung für die Mundpflege bereits nach vergleichsweise kurzer Dauer einer regelmäßigen Anwendung (bspw. ca. 2 - 4 Wochen) vorhandenen Zahnbelag aufzulösen und zu entfernen vermag.

Sogar Zahnstein lässt sich in überraschender Weise mit der Zusammensetzung zunächst aufweichen und schlussendlich entfernen.

Zusätzlich zeigt sich eine bemerkenswerte antibakterielle Wirkung der erfindungsgemäßen Zusammensetzung für die Mundpflege. Damit wirkt die Zusammensetzung nicht nur gegen die genannten Arten von Belägen auf Zahnoberflächen, sondern vermag darüber hinaus gegen Karies sowie Erkrankungen der Gingiva zu wirken.

Die erfindungsgemäße Zusammensetzung kann als homogenes (einphasiges) Spray oder Gel im Mundraum angewendet werden. Dadurch wird die Anwendung vereinfacht. Eine Zahnbürste ist dabei zum Auftragen der Zusammensetzung nicht zwingend erforderlich, kann aber nach Wunsch unterstützend benutzt werden. Eine Anwendung als Mundspülung, Paste oder dgl. ist alternativ möglich.

Die erfindungsgemäße Zusammensetzung kann vollständig aus natürlichen Inhaltsstoffen bestehen, wodurch eine wünschenswerte Transparenz gegenüber dem Verbraucher erreicht wird. Der Verbraucher muss sich nicht mit ihm nicht bekannten synthetischen Inhaltsstoffen und deren vermeintlichen Nachteilen und/oder unerwünschten Wirkungen auseinandersetzen. Die Inhaltsstoffe der erfindungsgemäßen Zusammensetzung sind für sich gesehen unbedenklich und wirken innerhalb des Mundraums in milder und verträglicher Art und Weise.

Darüber hinaus lässt sich die erfindungsgemäße Zusammensetzung vergleichsweise kostengünstig herstellen.

In einer vorteilhaften ersten Weiterbildung des Erfindungsgedankens beträgt der Gehalt an Pfefferminzöl 0,2 - 2 Massen-% (Ma.-%) an der erfindungsgemäßen Zusammensetzung. Alternativ kann ein Gehalt an Pfefferminzöl von 0,2 - 5 Ma.-%, insbesondere von 1,2 - 2 Ma.-%, bevorzugt sein.

Gemäß einer weiteren bevorzugten Ausführungsform beträgt der Gehalt von Rosmarinöl 0,2 - 2,5 Ma.-%, besonders bevorzugt 0,5 - 1,2 Ma.-%, an der Zusammensetzung. Alternativ kann ein Gehalt an Rosmarinöl von 0,2 - 0,5 Ma.-% bevorzugt sein.

Alternativ oder zusätzlich zu den vorgenannten Ausgestaltungen kann der Gehalt an Alkohol bevorzugt 15 - 35 Ma.-% an der Zusammensetzung betragen.

In einem weiteren vorteilhaften Ausführungsbeispiel weist die Zusammensetzung Niemöl, insbesondere mit einem Gehalt von 0,05 - 10 Ma.-%, und insbesondere mit einem Gehalt von 0,1 - 0,2 Ma.-%, auf.

In einer weiteren konkreten und bevorzugten Ausgestaltung weist die Zusammensetzung ferner Thymianöl, insbesondere mit einem Gehalt von 0,05 - 0,5 Ma.-%, und insbesondere mit einem Gehalt von 0,1 - 0,2 Ma.-%, auf.

Gemäß einer weiteren vorteilhaften Weiterbildung weist die Zusammensetzung alternativ oder zusätzlich Grapefruitkernextrakt, insbesondere mit einem Gehalt von 0,05 - 1,5 Ma.-%, und insbesondere mit einem Gehalt von 0,1 - 1,0 Ma.-%, auf.

Bevorzugt ist ferner eine Zusammensetzung, welche Traubenkernextrakt, insbesondere mit einem Gehalt von 0,05 - 1,5 Ma.-%, und insbesondere mit einem Gehalt von 0,1 - 1,0 Ma.-%, aufweist.

Gemäß einer weiteren vorteilhaften Weiterbildung weist die Zusammensetzung alternativ oder zusätzlich Nelkenöl, insbesondere mit einem Gehalt von 0,1 - 4,0 Ma.-%, und insbesondere mit einem Gehalt von 0,25 - 2,0 Ma.-%, auf.

In einer weiteren vorteilhaften Weiterbildung weist die Zusammensetzung alternativ oder zusätzlich Polyglyceryl-10 Decaoleat, insbesondere mit einem Gehalt von 5 - 15 Ma.-%, und insbesondere mit einem Gehalt von 10 Ma.-%, auf.

In einer weiteren vorteilhaften Weiterbildung weist die Zusammensetzung alternativ oder zusätzlich Polyglyceryl-10 Pentaoleat, insbesondere mit einem Gehalt von 5 - 15 Ma.-%, und insbesondere mit einem Gehalt von 10 Ma.-%, auf.

In einer weiteren vorteilhaften Weiterbildung weist die Zusammensetzung alternativ oder zusätzlich Polyethylenglykol (PEG), insbesondere PEG 3350, auf. Der Gehalt an PEG kann dabei vorzugsweise 5 - 20 Ma.-%, besonders bevorzugt 15 Ma.-%, betragen.

In einer weiteren vorteilhaften Ausgestaltung weist die Zusammensetzung alternativ oder zusätzlich Cyclomethicon, insbesondere mit einem Gehalt von 8 - 18 Ma.-%, und insbesondere mit einem Gehalt von 10 - 12,5 Ma.-%, auf,

In einem weiteren vorteilhaften Aspekt der Erfindung weist die Zusammensetzung alternativ oder zusätzlich Ceramid-III, insbesondere mit einem Gehalt von 7,5 - 12,5 Ma.-%, und insbesondere mit einem Gehalt von 10 Ma.-%, auf.

Als vorteilhaft erweist sich ferner eine Zusammensetzung, welche alternativ oder zusätzlich Butylstearat, insbesondere mit einem Gehalt von 5 - 30 Ma.-%, und insbesondere mit einem Gehalt von 10 - 25 Ma.-%, aufweist.

In einer weiteren vorteilhaften Weiterbildung weist die Zusammensetzung alternativ oder zusätzlich Planktonextrakt, bevorzugt mit einem Gehalt von 7,5 - 12,5 Ma.-%, besonders bevorzugt mit einem Gehalt von 10 Ma.-%, auf.

In einem weiteren vorteilhaften Aspekt der Erfindung weist die Zusammensetzung alternativ oder zusätzlich Lecithin, insbesondere mit einem Gehalt von 7,5 - 12,5 Ma.-%, und insbesondere mit einem Gehalt von 10 Ma.-%, auf.

Innerhalb der erfindungsgemäßen Zusammensetzung weist Alkohol Ethylalkohol, insbesondere vergällten Ethylalkohol, auf.

Ferner ist eine Ausgestaltung bevorzugt, in der das Wasser entionisiertes Wasser aufweist.

Die erfindungsgemäße Zusammensetzung erweist sich als besonders vorteilhaft, sofern sie als Zubereitung zum Sprühen oder als Spray oder als Mundspüllösung ausgebildet ist.

In einer Alternative erweist sich die Ausgestaltung als Lutschtablette, Pastille, Bonbon oder Kaugummi ebenfalls als vorteilhaft. Eine weitere besondere Ausgestaltung betrifft einen Haftstreifen zum Aufbringen auf die Zähne. Solche Haftstreifen sind, mit einem geeigneten Wirkstoff versehen, für sich gesehen bekannt, um die Aufhellung bzw. das Bleichen von Zähnen zu bewirken. Im Rahmen der vorliegenden Erfindung wird nun vorgeschlagen, die erfindungsgemäße Zusammensetzung mittels eines solchen Haftstreifens in möglichst dauerhaften Kontakt mit den Zahnoberflächen zu bringen.

Alternativ, aber ebenfalls bevorzugt, kann die Zusammensetzung als Gel oder gelartig ausgebildet ist, insbesondere wobei die Zusammensetzung zumindest ein Geliermittel, vorzugsweise Polyacrylsäure und/oder Xanthan, aufweist.

Hierbei kann die Zusammensetzung gemäß einer weiteren konkreten Weiterbildung als Geliermittel Polyacrylsäure mit einem Gehalt von 0,2 - 1,0 Ma.-%, insbesondere 0,5 Ma.-%, aufweisen.

Ferner, nach einem weiteren nicht einschränkenden Ausführungsbeispiel, weist die Zusammensetzung Triethanolamin mit einem Gehalt von 0,2 - 1,0 Ma.-%, insbesondere 0,5 Ma.-%, auf. Triethanolamin kann dabei einerseits als Geliermittel für eine Ausgestaltung als Gel dienen. Andererseits kann Triethanolamin auch zur pH-Einstellung und/oder zur Viskositätseinstellung in Ausgestaltungen der Zusammensetzung als Spray, Spülung oder dgl. dienen.

Im Rahmen weiterer bevorzugter optionaler Ausgestaltungen weist die Zusammensetzung alternativ oder zusätzlich Oreganoöl und/oder Tamanuöl und/oder Grüner Tee Extrakt auf.

Im Rahmen der Erfindung wird die Verwendung der erfindungsgemäßen Zusammensetzung zur Mund- und/oder Zahnpflege und/oder zur oralen Prophylaxe vorgeschlagen.

Ferner wird im Rahmen der Erfindung die Verwendung der erfindungsgemäßen Zusammensetzung zur Lösung und/oder Entfernung von extrazellulären polymeren Substanzen (EPS) auf Zahnoberflächen, und/oder von Zahnbelag (Plaque), und/oder von Zahnstein vorgeschlagen. Des Weiteren wird eine Verwendung zur Modifikation der Pellikelschicht auf Zahnoberflächen vorgeschlagen. Die genannten Verwendungen können dabei ggf. auch gleichzeitig realisiert werden.

Angegeben ist schließlich ein erfindungsgemäßes Verfahren zur Lösung und/oder Entfernung von extrazellulären polymeren Substanzen (EPS) auf Zahnoberflächen, und/oder von Zahnbelag (Plaque), und/oder von Zahnstein, wobei das Verfahren die Anwendung der erfindungsgemäßen Zusammensetzung im Mundraum, und insbesondere auf Zahnoberflächen, aufweist.

Ein weiteres erfindungsgemäßes Verfahren betrifft die Modifikation der Pellikelschicht auf Zahnoberflächen, wobei das Verfahren die Anwendung der erfindungsgemäßen Zusammensetzung im Mundraum, und insbesondere auf Zahnoberflächen, aufweist.

Die genannten Verfahren lassen sich dabei ggf. auch gleichzeitig durchführen.

Die Vorteile der erfindungsgemäßen Verwendungen bzw. der erfindungsgemäßen Verfahren gegenüber bekannten Gegenständen der jeweiligen Gattung ergeben sich dabei bereits aus den obigen Ausführungen zu Wesen und Vorteilen der erfindungsgemäßen Zusammensetzung, auf welche zur Vermeidung von Wiederholungen vollumfänglich verwiesen wird.

Des Weiteren ergeben sich durch die obigen Erläuterungen vorteilhafter Ausgestaltungen und Weiterbildungen der erfindungsgemäßen Zusammensetzung gleichzeitig auch vorteilhafte Ausgestaltungen und Weiterbildungen der erfindungsgemäßen Verwendungen bzw. der erfindungsgemäßen Verfahren. Dabei lassen sich Merkmale verschiedener vorteilhafter Ausgestaltungen der Zusammensetzung auch innerhalb vorteilhafter Weiterbildungen der Verwendungen bzw. der Verfahren kombinieren.

### Beispiele

Ausführungsbeispiele der Erfindung, welche die Erfindung jedoch nicht auf die dargestellten Beispiele einschränken, werden im Rahmen der folgenden tabellarischen Aufstellungen von beispielhaften erfindungsgemäßen Zusammensetzungen gezeigt. Diese beispielhaften Zusammensetzungen geben gleichzeitig - zusätzlich oder ergänzend zum Gegenstand der nachgeordneten Ansprüche - besonders bevorzugte Ausführungsformen der erfindungsgemäßen Zusammensetzung wieder.

Sämtliche Zahlenwerte verstehen sich dabei als Massen-%, bezogen auf die gesamte Masse der Zusammensetzung.

Die unten aufgeführten Beispielzusammensetzungen sind i.A. als Spray bzw. Lösung ausgebildet, wobei8 jedoch die Zusammensetzungen 7 und 8 durch die Zugabe zumindest eines Geliermittels als Gel bzw. gelartig ausgebildet sind.

| | Beispiel 0 | Beispiel 1B |
|---|---|---|
| Wasser (entionisiert) | 72,25 | 53 |
| Ethylalkohol (vergällt) | 25 | 35 |
| Pfefferminzöl | 1,2 | 5 |
| Rosmarinöl | 1,2 | 5 |
| Grapefruitkernextrakt | 0,1 | - |
| Traubenkernextrakt | 0,1 | - |
| Thymianöl | 0,05 | - |
| Niemöl | 0,1 | - |
| Nelkenöl | - | 2 |

| | Beispiel 1 | Beispiel 2 | Beispiel 3 |
|---|---|---|---|
| Wasser (entionisiert) | 55,6 | 35,6 | 86,3 |
| Ethylalkohol (vergällt) | 35 | 60 | 12,5 |
| Pfefferminzöl | 5 | 5 | 0,2 |
| Rosmarinöl | 2 | 2 | 0,2 |
| Grapefruitkernextrakt | 1 | 1 | 0,2 |
| Traubenkernextrakt | 1 | 1 | 0,2 |
| Thymianöl | 0,2 | 0,2 | 0,2 |
| Niemöl | 0,2 | 0,2 | 0,2 |

| | Beispiel 4B | **Beispiel 4** |
|---|---|---|
| Wasser (entionisiert) | 62,6 | 64,5 |
| Ethylalkohol (vergällt) | 35 | 35 |
| Pfefferminzöl | 1,2 | 0,1 |
| Rosmarinöl | 1,2 | 0,1 |
| Grapefruitkernextrakt | - | 0,05 |
| Traubenkernextrakt | - | 0,05 |
| Thymianöl | - | 0,1 |
| Niemöl | - | 0,1 |

| | Beispiel 4C | Beispiel 5 | Beispiel 6 |
|---|---|---|---|
| Wasser (entionisiert) | 64,5 | 38,8 | 63,8 |
| Ethylalkohol (vergällt) | 35 | 60 | 35 |
| Pfefferminzöl | 0,1 | 1 | 1 |
| Rosmarinöl | 0,1 | 0,1 | 0,1 |
| Grapefruitkernextrakt | 0,1 | 0,1 | 0,1 |
| Traubenkernextrakt | 0,1 | - | - |
| Thymianöl | 0,05 | - | - |
| Niemöl | 0,05 | - | - |

| | Beispiel 7 | Beispiel 8 |
|---|---|---|
| Wasser (entionisiert) | 60,1 | 34,6 |
| Ethylalkohol (vergällt) | 35 | 60 |
| Pfefferminzöl | 1 | 5 |
| Rosmarinöl | 0,5 | 2 |
| Grapefruitkernextrakt | 1 | 1 |
| Traubenkernextrakt | 1 | 1 |
| Thymianöl | 0,2 | 0,2 |
| Niemöl | 0,2 | 0,2 |
| Polyacrylsäure | 0,5 | 0,5 |
| Triethanolamin (TEA) | 0,5 | 0,5 |

| | Beispiel 11 | **Beispiel 12** | Beispiel 13 |
|---|---|---|---|
| Wasser (entionisiert) | 62,6 | 62,6 | 50,1 |
| Ethylalkohol (vergällt) | 25 | 25 | 25 |
| Pfefferminzöl | 1,2 | 1,2 | 1,2 |
| Rosmarinöl | 1,2 | 1,2 | 1,2 |
| Polyglyceryl-10 Decaoleat | - | 10 | - |
| Polyglyceryl-10 Pentaoleat | 10 | - | - |
| Cyclomethicon | - | - | 12,5 |

| | **Beispiel 14** | Beispiel 15 | Beispiel 16 |
|---|---|---|---|
| Wasser (entionisiert) | 57,6 | 62,6 | 62,6 |
| Ethylalkohol (vergällt) | 25 | 25 | 25 |
| Pfefferminzöl | 1,2 | 1,2 | 1,2 |
| Rosmarinöl | 1,2 | 1,2 | 1,2 |
| Polyethylenglykol (PEG) 3350 | 15 | - | - |
| Planktonextrakt | - | - | 10 |
| Cyclomethicon | - | 10 | - |

| | Beispiel 19 | Beispiel 20 |
|---|---|---|
| Wasser (entionisiert) | 47,6 | 59,75 |
| Ethylalkohol (vergällt) | 25 | 25 |
| Pfefferminzöl | 1,2 | 2,5 |
| Rosmarinöl | 1,2 | 2,5 |
| Nelkenöl | - | 0,25 |
| Butylstearat | 25 | 10 |

### Versuche

Im Folgenden werden Versuchsergebnisse erörtert, welche mit ausgewählten Zusammensetzungen aus dem vorhergehenden Abschnitt "Beispiele" erhalten worden sind. Dabei wird ergänzend auch auf die angefügten Figuren Bezug genommen, welche die erhaltenen Ergebnisse in einer Gegenüberstellung mit Kontrollaufnahmen ohne vorherige Anwendung einer erfindungsgemäßen Zusammensetzung darstellen.

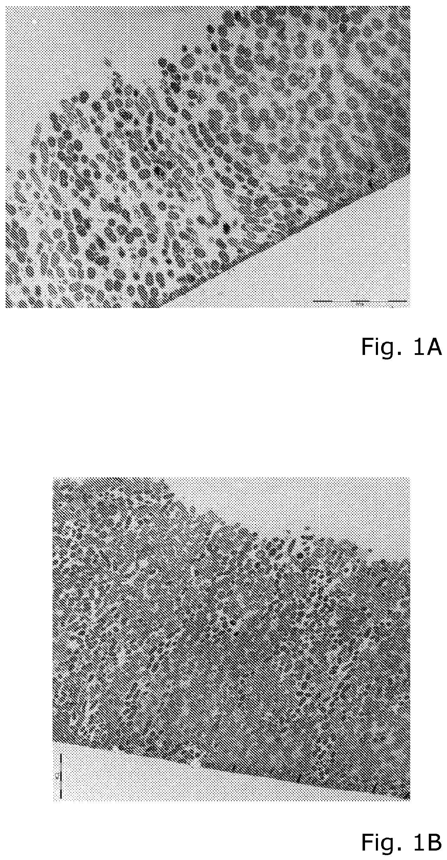

Die **Figuren 1 bis 7** zeigen elektronenmikroskopisch erhaltene Aufsicht- bzw. Schnittbilder von einheitlich hergestellten Prüfkörpern aus bovinem Zahnschmelz (Zahnschmelzprüfkörper), welche von Versuchspersonen in der Mundhöhle (in vivo) getragen worden sind. Die jeweiligen Versuchsbedingungen werden dabei anhand der konkreten Figuren erläutert. Die Figuren 1B, 2B, 3B, 4B und 6B zeigen dabei jeweils zum Vergleich Kontrollaufnahmen nach denselben jeweiligen Versuchsbedingungen, jedoch ohne Anwendung einer erfindungsgemäßen Lösung.

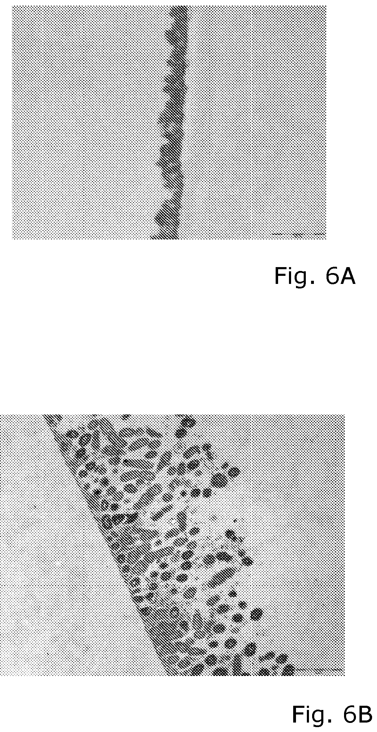

In Bezug auf die Querschnittsaufnahmen der Zahnschmelzprüfkörper gemäß Fig. 1 und 6 sei darauf hingewiesen, dass der Zahnschmelz jeweils im Rahmen der Probenvorbereitung für die transmissionselektronenmikroskopische Analyse herausgelöst wurde und daher an der Basis des Biofilms nicht mehr zu erkennen ist.

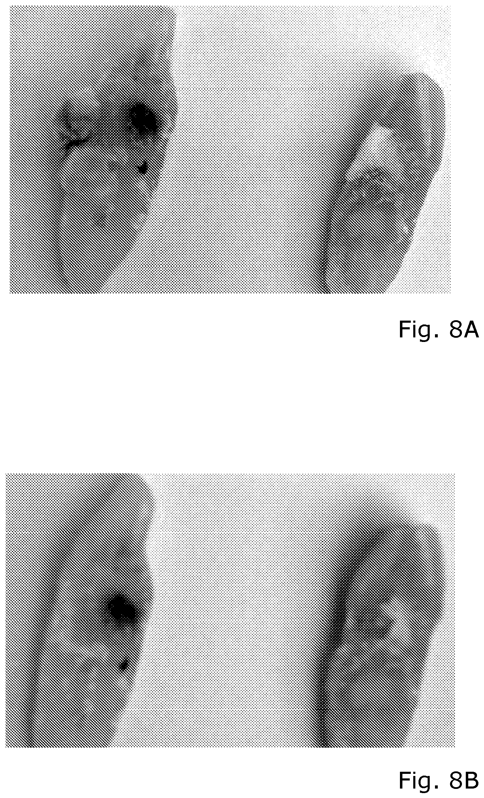

Die **Figuren 8A und 8B** zeigen fotografische Aufnahmen zweier extrahierter humaner Zähne mit urspünglich stark ausgeprägtem Zahnsteinbelag vor (Fig. 8A) und nach (Fig. 8B) der Anwendung einer erfindungsgemäßen Lösung.

### Versuch 1: Beispiel für das Herauslösen der Biofilmmatrix (der extrazellulären polymeren Substanzen, EPS):

Innerhalb von 48h bildet sich auf der Zahnschmelzoberfläche unter Mundhöhlenbedingungen ein dichter bakterieller Biofilm aus, sofern jegliche Mundhygienemaßnahmen unterlassen werden (vgl. **Fig. 1B**, welche eine entsprechende Kontrollaufnahme nach 48h ohne Anwendung einer erfindungsgemäßen Zusammensetzung darstellt).

Transmissionselektronenmikroskopisch lassen sich im Querschnitt durch den Biofilm überwiegend kugelförmige Bakterien identifizieren. Der Biofilm zeigt eine dichte kompakte Ultrastruktur, zwischen den Bakterien sind die Hohlräume von interbakterieller Matrix aufgefüllt.

Der Zahnschmelz wurde im Rahmen der Probenvorbereitung für die transmissionselektronenmikroskopische Analyse herausgelöst und ist daher an der Basis des Biofilms nicht mehr zu erkennen.

Wird dieser 48h-Biofilm gemäß Fig. 1B außerhalb der Mundhöhle für einen Zeitraum von 1h in einer erfindungsgemäßen Lösung mit der Zusammensetzung von **Beispiel 4** (nicht: 4B oder 4C) aus dem vorstehenden Abschnitt "Beispiele" exponiert, so werden die äußeren Bakterienlagen des Biofilms abgelöst, und die interbakterielle Matrix wird herausgelöst. Es resultiert im transmissionselektronenmikroskopischen Bild eine "aufgelockerte" Ultrastruktur des Biofilms.

Dieses Ergebnis wird durch die entsprechende Aufnahme gemäß **Fig. 1A** verdeutlicht.

### Versuch 2: Beispiel für das Herauslösen der Biofilmmatrix:

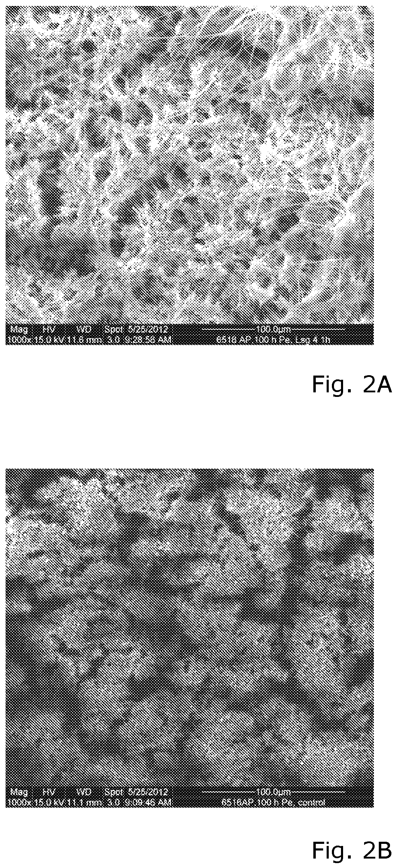

Nach 100-stündiger Exposition in der Mundhöhle (ohne zwischenzeitliche Mundhygienemaßnahmen) bildet sich auf der Oberfläche des Zahnschmelzprüfkörpers bzw. auf der Schmelzoberfläche ein dichter Belag aus adhärenten Mikroorganismen. Im rasterelektronenmikroskopischen Bild bzw. in der rasterelektronenmikroskopischen Aufsicht stellt sich dieser Biofilm als bakterieller Multilayer und dichte Oberflächenbedeckung dar, die von trocknungsbedingten Rissbildungen durchzogen wird. Zum Teil sind an der Oberfläche des Biofilms einzelne faden-, stäbchen- und kokkenförmige Bakterien zu identifizieren. (vgl. **Fig. 2B****,** welche eine Kontrollaufnahme nach diesen Bedingungen und ohne Anwendung einer erfindungsgemäßen Zusammensetzung darstellt).

Wird dieser Biofilm außerhalb der Mundhöhle für 1h in der Lösung gemäß **Beispiel 4** exponiert, so resultiert ein aufgelockertes, zerklüftetes netzartiges Oberflächengefüge des Biofilms in der rasterelektronenmikroskopischen Aufsicht. Die faden-, stäbchen- und kugelförmigen Bakterien sind gut zu detektieren, die interbakterielle Matrix ist herausgelöst. Dieses Ergebnis wird ergänzend durch die entsprechende Aufnahme des betreffenden Prüfkörpers gemäß **Fig. 2A** verdeutlicht.

### Versuch 3: Beispiel für verringerte Biofilm-Neubildung:

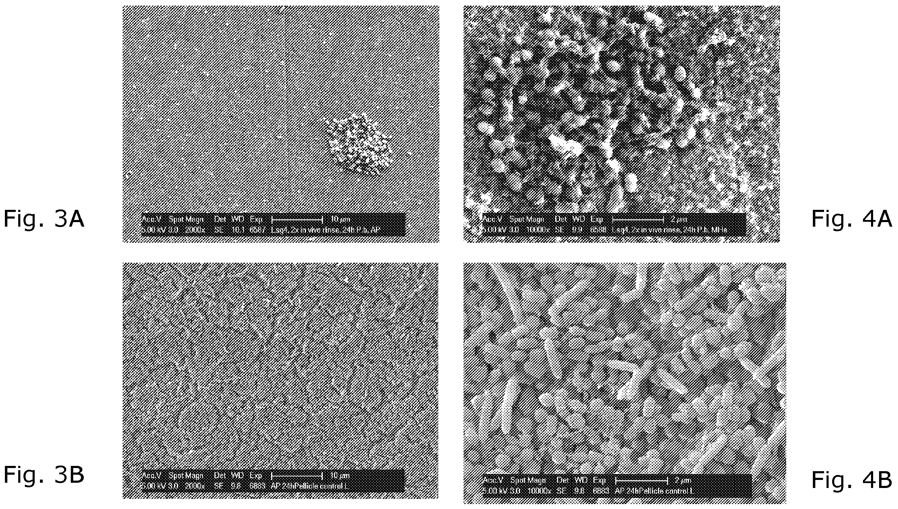

Innerhalb von 24h ohne zwischenzeitliche Mundhygiene bildet sich auf der Zahnschmelzoberfläche unter Mundhöhlenbedingungen ein dichter Biofilm ("Bakterienrasen") aus. In der rasterelektronenmikroskopischen Aufsicht imponieren bei höherer Vergrößerung kugel- und stäbchenförmige Bakterien, die als mehrlagiger Biofilm die Schmelzoberfläche bedecken und vollständig maskieren. Dies wird durch die entsprechenden Kontrollaufnahmen gemäß **Fig. 3B** und in **Fig. 4B** dargestellt. Fig. 4B zeigt dabei im Vergleich zu Fig. 3B eine fünffach vergrößerte Aufnahme. Ein entsprechendes Größenverhältnis gilt ebenfalls für den Vergleich der Fig. 4A und 3A (s. folgender Absatz).

Wird während der 24-stündigen Exposition der Schmelzprüfkörper in der Mundhöhle im Abstand von 12h zweimal für jeweils 30s mit der Lösung gemäß **Beispiel 4** gespült, so ist die Biofilmneubildung deutlich reduziert. Es lassen sich nur einzelne Inseln von adhärenten Bakterien, jedoch kein geschlossener Biofilm, auf der Schmelzoberfläche in der rasterelektronenmikroskopischen Aufsicht darstellen. Die Zahnschmelzoberfläche ist von einer granulär erscheinenden Proteinschicht (Pellikelschicht) bedeckt. Diese Pellikel trägt zur partiellen Maskierung der Zahnschmelzoberfläche bei. Dieses besonders vorteilhafte Ergebnis wird ergänzend durch die entsprechenden Aufnahmen eines entsprechend behandelten Prüfkörpers gemäß **Fig. 3A und 4A** verdeutlicht.

### Versuch 4: Beispiel für massiv reduzierte Biofilmneubildung und Pellikelmodifikation :

Nach zweimaliger Spülung mit einer Lösung mit der Zusammensetzung von **Beispiel 12** aus dem vorhergehenden Abschnitt "Beispiele" über jeweils 30s im Abstand von 12h ist die 24h-Biofilmbildung unter Mundhöhlenbedingungen nahezu vollständig unterbunden (inhibiert). Nach 24-stündiger intraoraler Exposition ist die Schmelzoberfläche unter dem Einfluss der zweimaligen Spülung mit der Lösung nach Beispiel 12 von einer Pellikelschicht, die sich im rasterelektronenmikroskopischen Bild netzartig granulär darstellt, partiell maskiert. Auf der Pellikel lassen sich nur (sehr vereinzelt) einzelne adhärente Bakterien detektieren.

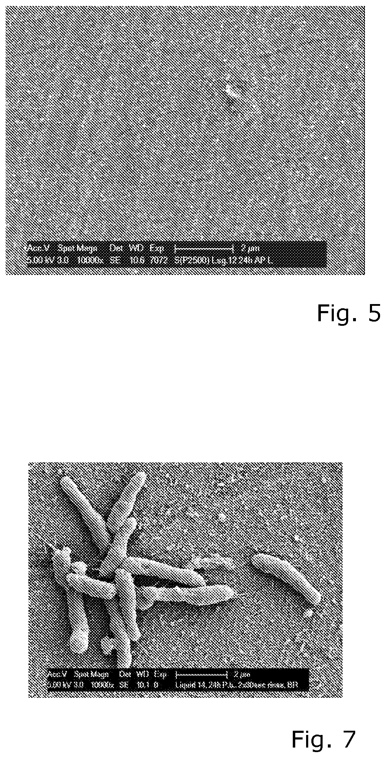

Dieses Ergebnis wird ergänzend durch die Aufnahme eines entsprechend behandelten Prüfkörpers gemäß **Fig. 5** verdeutlicht.

Als vergleichbare Kontrollaufnahme ohne Anwendung einer erfindungsgemäßen Lösung dient hierbei erneut **Fig. 4B****.**

### Versuch 5: Beispiel für verringerte Biofilmneubildung und modifizierte Pellikelbildung:

Auch nach zweimaliger Spülung mit einer Lösung mit der Zusammensetzung von **Beispiel 14** aus dem vorhergehenden Abschnitt "Beispiele" für jeweils 30s ist die 24h-Biofilmbildung unter Mundhöhlenbedingungen deutlich reduziert im Vergleich zur Kontrolle (24h-Biofilmbildung ohne zwischenzeitliches Zähneputzen, vgl. Kontrollaufnahme gemäß **Fig. 4B**). Es lassen sich rasterelektronenmikroskopisch unter dem Einfluss der zweimaligen Spülung mit der Lösung nach Beispiel 14 nur vereinzelte Aggregate von adhärenten Bakterien detektieren. Die Zahnschmelzoberfläche ist von einer sehr dichten und homogenen Pellikelschicht bedeckt, was ergänzend durch die Aufnahme einer entsprechend behandelten Prüfkörperoberfläche gemäß **Fig. 7** belegt wird. Zum Vergleich hierzu dient erneut die Kontrollaufnahme gemäß **Fig. 4B****.**

Des Weiteren stellt sich die oben erläuterte, durch Anwendung der erfindungsgemäßen Lösung nach Beispiel 14 erhaltene homogene Pellikelschicht transmissionselektronenmikroskopisch im Querschnittspräparat als elektronendichte, granuläre Oberflächenbedeckung dar. Dies wird durch die entsprechende Aufnahme des Querschnittspräparats gemäß **Fig. 6A** verdeutlicht.

Die entsprechende Kontrollaufnahme gemäß **Fig. 6B** zeigt den Querschnitt durch einen Kontrollkörper, der unter den gleichen Bedingungen, jedoch ohne Anwendung einer erfindungsgemäßen Lösung, erhalten worden ist. Im Vergleich mit der homogenen Pellikelschicht gemäß Fig. 6A zeigt sich hier bei Fig. 6B die Entstehung eines mehrlagigen bakteriellen Biofilms.

### Versuch 6: Beispiel für die Lösung und für die Entfernung von Zahnstein:

Im Rahmen dieses Versuchs wurden zwei extrahierte Zähne mit starkem Zahnsteinbelag mit der Lösung gemäß **Beispiel 14** behandelt (24h Exposition). Dabei kommt es zu einem "auflösen" des Verbundes zwischen Zahnstein und Zahnhartsubstanz; die Haftung des Zahnsteines an der Zahnoberfläche ist reduziert. Nach dem Einwirken der Lösung nach Beispiel 14 lässt sich der Zahnstein daher mit einer Zahnbürste von der Zahnoberfläche nahezu vollständig entfernen / abbürsten.

Normalerweise kann der Zahnstein nur unter dem Einfluss erheblicher mechanischer Kräfte mit einem Scaler oder unter Einsatz von ultraschallbetriebenen Zahnsteinentfernungsgeräten von der Zahnoberfläche abgetragen werden. Die **Fig. 8A und 8B** zeigen Aufnahmen der extrahierten Zähne, einerseits mit starkem Zahnsteinbelag vor der Exposition in Lösung 14 **(****Fig. 8A****)** und andererseits nach der Durchführung des vorstehend erörterten Versuchs **(****Fig. 8B****).**

## Patentansprüche

1. Zusammensetzung für die Mundpflege, wobei die Zusammensetzung eine wässrige Zusammensetzung ist und neben Wasser aufweist (in Massen-%):
| | |
|---|---|
| Pfefferminzöl: | 0,05 - 10% |
| Rosmarinöl: | 0,05 - 5% |
| Alkohol, nämlich Ethylalkohol: | 5 - 75% |

2. Zusammensetzung nach Anspruch 1, **dadurch gekennzeichnet, dass** der Gehalt an Pfefferminzöl 0,2 - 5 Massen-% (Ma.-%), insbesondere 1,2 - 2 Ma.-%, beträgt, und/oder
dass der Gehalt an Rosmarinöl 0,2 - 2,5 Ma.-%, und insbesondere 0,5 - 1,2 Ma.%, beträgt, und/oder
dass der Gehalt an Ethylalkohol, insbesondere an vergälltem Ethylalkohol, 15 - 35 Ma.-% beträgt.

3. Zusammensetzung nach Anspruch 1 oder 2, **dadurch gekennzeichnet, dass** die Zusammensetzung Niemöl, insbesondere mit einem Gehalt von 0,05 - 10 Ma.-%, und insbesondere mit einem Gehalt von 0,1 - 0,2 Ma.-%, aufweist.

4. Zusammensetzung nach einem der Ansprüche 1 bis 3, **dadurch gekennzeichnet, dass** die Zusammensetzung Thymianöl, insbesondere mit einem Gehalt von 0,05 - 0,5 Ma.-%, und insbesondere mit einem Gehalt von 0,1 - 0,2 Ma.-%, aufweist.

5. Zusammensetzung nach einem der Ansprüche 1 bis 4, **dadurch gekennzeichnet, dass** die Zusammensetzung Grapefruitkernextrakt, insbesondere mit einem Gehalt von 0,05 - 1,5 Ma.-%, und insbesondere mit einem Gehalt von 0,1 - 1,0 Ma.-%, aufweist, und/oder
dass die Zusammensetzung Traubenkernextrakt, insbesondere mit einem Gehalt von 0,05 - 1,5 Ma.-%, und insbesondere mit einem Gehalt von 0,1 - 1,0 Ma.-%, aufweist.

6. Zusammensetzung nach einem der Ansprüche 1 bis 5, **dadurch gekennzeichnet, dass** die Zusammensetzung Nelkenöl, insbesondere mit einem Gehalt von 0,1 - 4,0 Ma.-%, und insbesondere mit einem Gehalt von 0,25 - 2,0 Ma.-%, aufweist.

7. Zusammensetzung nach einem der Ansprüche 1 bis 6, **dadurch gekennzeichnet, dass** die Zusammensetzung Polyglyceryl-10 Decaoleat, insbesondere mit einem Gehalt von 5 - 15 Ma.-%, und insbesondere mit einem Gehalt von 10 Ma.-%, aufweist, und/oder
dass die Zusammensetzung Polyglyceryl-10 Pentaoleat, insbesondere mit einem Gehalt von 5 - 15 Ma.-%, und insbesondere mit einem Gehalt von 10 Ma.-%, aufweist.

8. Zusammensetzung nach einem der Ansprüche 1 bis 7, **dadurch gekennzeichnet, dass** die Zusammensetzung Polyethylenglykol (PEG), insbesondere PEG 3350, insbesondere mit einem Gehalt von 5 - 20 Ma.-%, und insbesondere mit einem Gehalt von 15 Ma.-%, aufweist.

9. Zusammensetzung nach einem der Ansprüche 1 bis 8, **dadurch gekennzeichnet, dass** die Zusammensetzung als Zubereitung zum Sprühen oder als Spray oder als Mundspüllösung ausgebildet ist.

10. Zusammensetzung nach einem der Ansprüche 1 bis 9, **dadurch gekennzeichnet, dass** die Zusammensetzung als Gel oder gelartig ausgebildet ist, insbesondere wobei die Zusammensetzung zumindest ein Geliermittel, vorzugsweise Polyacrylsäure und/oder Xanthan, aufweist, und insbesondere wobei
die Zusammensetzung Polyacrylsäure mit einem Gehalt von 0,2 - 1,0 Ma.-%, insbesondere 0,5 Ma.-%, aufweist.

11. Zusammensetzung nach einem der Ansprüche 1 bis 10, **dadurch gekennzeichnet, dass** die Zusammensetzung Triethanolamin mit einem Gehalt von 0,2 - 1,0 Ma.-%, insbesondere 0,5 Ma.-%, aufweist.

12. Zusammensetzung nach einem der Ansprüche 1 bis 11 zur Verwendung zur Lösung und/oder Entfernung von
extrazellulären polymeren Substanzen (EPS) auf Zahnoberflächen, und/oder Zahnbelag (Plaque),
und/oder Zahnstein.

13. Zusammensetzung nach einem der Ansprüche 1 bis 11 zur Anwendung in einem Verfahren zur Lösung und/oder Entfernung von
extrazellulären polymeren Substanzen (EPS) auf Zahnoberflächen, und/oder Zahnbelag (Plaque),
und/oder Zahnstein,
wobei das Verfahren die Anwendung der Zusammensetzung im Mundraum, und insbesondere auf Zahnoberflächen, aufweist.

14. Zusammensetzung nach einem der Ansprüche 1 bis 11 zur Verwendung
zur Modifikation der Pellikelschicht auf Zahnoberflächen, oder
zur Mund- und/oder Zahnpflege und/oder zur oralen Prophylaxe.

15. Zusammensetzung nach einem der Ansprüche 1 bis 11 zur Anwendung in einem Verfahren zur Modifikation der Pellikelschicht auf Zahnoberflächen, wobei das Verfahren die Anwendung der Zusammensetzung im Mundraum, und insbesondere auf Zahnoberflächen, aufweist.

## Claims

1. Composition for oral care, wherein the composition is an aqueous composition, and, besides water, comprises (in weight percentages):
| | |
|---|---|
| Peppermint oil: | 0.05 - 10% |
| Rosemary oil: | 0.05 - 5% |
| Alcohol, namely ethyl alcohol: | 5 - 75% |

2. Composition according to claim 1, **characterised in that** the content of peppermint oil is 0.2 - 5 weight percentages (wt.%), in particular 1.2 - 2 wt.%, and/or
that the content of rosemary oil is 0.2 - 2.5 wt.%, and particularly 0.5 -1.2 wt.%, and/or
that the content of ethyl alcohol, particularly denatured ethyl alcohol, is 15 - 35 wt.%.

3. Composition according to claim 1 or 2, **characterised in that** the composition comprises neem oil, in particular with a content of 0.05 - 10 wt.%, and particularly with a content of 0.1 - 0.2 wt.%.

4. Composition according to one of the claims 1 to 3, **characterised in that** the composition comprises thyme oil, in particular with a content of 0.05 - 0,5 wt.%, and particularly with a content of 0.1 - 0.2 wt.%.

5. Composition according to one of the claims 1 to 4, **characterised in that** the composition comprises grapefruit seed extract, in particular with a content of 0.05 - 1.5 wt.%, and particularly with a content of 0.1 - 1.0 wt.%, and/or
that the composition comprises grape seed extract, in particular with a content of 0.05 - 1.5 wt.%, and particularly with a content of 0.1 - 1.0 wt.%.

6. Composition according to one of the claims 1 to 5, **characterised in that** the composition comprises clove oil, in particular with a content of 0.1 - 4.0 wt.%, and particularly with a content of 0.25 - 2.0 wt.%.

7. Composition according to one of the claims 1 to 6, **characterised in that** the composition comprises polyglyceryl-10 decaoleate, in particular with a content of 5 - 15 wt.%, and particularly with a content of 10 wt.%, and/or
that the composition comprises polyglyceryl-10 pentaoleate, in particular with a content of 5 - 15 wt.%, and particularly with a content of 10 wt.%.

8. Composition according to one of the claims 1 to 7, **characterised in that** the composition comprises polyethylene glycol (PEG), in particular PEG 3350, in particular with a content of 5 - 20 wt %, and particularly with a content of 15 wt%.

9. Composition according to one of the claims 1 to 8, **characterised in that** the composition is configured as a preparation for spraying or as a spray or as a mouth rinsing solution.

10. Composition according to one of the claims 1 to 9, **characterised in that** the composition is configured as a gel or gel-like composition, in particular wherein the composition comprises at least one gelling agent, preferably polyacrylic acid and/or xanthan, and in particular wherein
the composition comprises polyacrylic acid with a content of 0.2 - 1.0 wt %, particularly 0.5 wt %.

11. Composition according to one of the claims 1 to 10, **characterised in that** the composition comprises triethanolamine with a content of 0.2 - 1.0 wt %, in particular 0.5 wt %.

12. Composition according to one of the claims 1 to 11 for use in the dissolution and/or the removal of
extracellular polymeric substances (EPS) on tooth surfaces,
and/or dental plaque (plaque),
and/or tartar.

13. Composition according to one of the claims 1 to 11 for use in a method for the dissolution and/or the removal of
extracellular polymeric substances (EPS) on tooth surfaces,
and/or dental plaque (plaque)
and/or tartar,
wherein the method comprises the application of said composition in the oral cavity, and particularly on tooth surfaces.

14. Composition according to one of the claims 1 to 11 for use
in the modification of the pellicle layer on tooth surfaces, or
in oral and/or dental care and/or in oral prophylaxis.

15. Composition according to one of the claims 1 to 11 for use in a method for the modification of the pellicle layer on tooth surfaces, wherein the method comprises the application of said composition in the oral cavity, and particularly on tooth surfaces.

## Revendications

1. Composition pour l'hygiène buccale, dans laquelle la composition est une composition aqueuse et présente outre de l'eau (en % en masse) :
| | |
|---|---|
| huile de menthe poivrée : | 0,05 à 10 % |
| huile de romarin : | 0,05 à 5 % |
| alcool, à savoir alcool éthylique : | 5 à 75 %. |

2. Composition selon la revendication 1, **caractérisée en ce que** la teneur en huile de menthe poivrée est de 0,2 à 5 % en masse, notamment 1,2 à 2 % en masse, et/ou
**que** la teneur en huile de romarin est de 0,2 à 2,5 % en masse, et notamment 0,5 à 1,2 % en masse, et/ou
**que** la teneur en alcool éthylique, notamment en alcool éthylique dénaturé, est de 15 à 35 % en masse.

3. Composition selon la revendication 1 ou 2, **caractérisée en ce que** la composition présente de l'huile de neem, notamment avec une teneur de 0,05 à 10 % en masse, et notamment avec une teneur de 0,1 à 0,2 % en masse.

4. Composition selon une des revendications 1 à 3, **caractérisée en ce que** la composition présente de l'huile de thym, notamment avec une teneur de 0,05 à 0,5 % en masse, et notamment avec une teneur de 0,1 à 0,2 % en masse.

5. Composition selon une des revendications 1 à 4, **caractérisée en ce que** la composition présente de l'extrait de pépin de pamplemousse, notamment avec une teneur de 0,05 à 1,5 % en masse, et notamment avec une teneur de 0,1 à 1,0 % en masse, et/ou
**que** la composition présente de l'extrait de pépin de raisin, notamment avec une teneur de 0,05 à 1,5 % en masse, et notamment avec une teneur de 0,1 à 1,0 % en masse.

6. Composition selon une des revendications 1 à 5, **caractérisée en ce que** la composition présente de l'huile de clou de girofle, notamment avec une teneur de 0,1 à 4,0 % en masse, et notamment avec une teneur de 0,25 à 2,0 % en masse.

7. Composition selon une des revendications 1 à 6, **caractérisée en ce que** la composition présente du décaoléate de polyglycéryle-10, notamment avec une teneur de 5 à 15 % en masse, et notamment avec une teneur de 10 % en masse, et/ou
**que** la composition présente du pentaoléate de polyglycéryle-10, notamment avec une teneur de 5 à 15 % en masse, et notamment avec une teneur de 10 % en masse.

8. Composition selon une des revendications 1 à 7, **caractérisée en ce que** la composition présente du polyéthylènegycol (PEG), notamment PEG 3350, notamment avec une teneur de 5 à 20 % en masse, et notamment avec une teneur de 15 % en masse.

9. Composition selon une des revendications 1 à 8, **caractérisée en ce que** la composition est réalisée comme préparation pour pulvérisation ou comme aérosol ou comme solution de rinçage buccal.

10. Composition selon une des revendications 1 à 9, **caractérisée en ce que** la composition est réalisée comme gel ou à la manière d'un gel, notamment dans laquelle la composition présente au moins un gélifiant, de préférence de l'acide polyacrylique et/ou du xanthane, et notamment dans laquelle la composition présente de l'acide polyacrylique avec une teneur de 0,2 à 1,0 % en masse, notamment de 0,5 % en masse.

11. Composition selon une des revendications 1 à 10, **caractérisée en ce que** la composition présente de la triéthanolamine avec une teneur de 0,2 à 1,0 % en masse, notamment de 0,5 % en masse.

12. Composition selon une des revendications 1 à 11 pour l'utilisation pour la dissolution et/ou l'élimination de
substances polymères extracellulaires (EPS) sur des surfaces dentaires, et/ou plaque dentaire
et/ou tartre.

13. Composition selon une des revendications 1 à 11 pour l'application dans un procédé pour la dissolution et/ou l'élimination de
substances polymères extracellulaires (EPS) sur des surfaces dentaires, et/ou plaque dentaire
et/ou tartre,
dans laquelle le procédé présente l'application de la composition dans la cavité buccale, et notamment sur des surfaces dentaires.

14. Composition selon une des revendications 1 à 11 pour l'utilisation
pour la modification de la couche de pellicule acquise sur des surfaces dentaires, ou
pour l'hygiène buccale et/ou dentaire et/ou pour la prophylaxie orale.

15. Composition selon une des revendications 1 à 11 pour l'application dans un procédé pour la modification de la couche de pellicule acquise sur des surfaces dentaires, dans laquelle le procédé présente l'application de la composition dans la cavité buccale, et notamment sur des surfaces dentaires.
